# EUROPÄISCHE PATENTANMELDUNG

(11) **EP 0 955 063 A1**
(43) Veröffentlichungstag der Anmeldung: **10.11.1999**
(21) Anmeldenummer: 99106023.7
(22) Anmeldetag: 25.03.1999
(51) Int. Cl.: A61K 47/34

(54) **Verwendung von wässrigen Zusammensetzungen in subkutanen oder intramuskulär zu verabreichenden Arzneimittel**

(30) Priorität: 01.04.1998 DE 19814513
(71) Anmelder: BASF AKTIENGESELLSCHAFT, 67056 Ludwigshafen (DE)
(72) Erfinder: Ruchatz, Folker, Dr., 67433 Neustadt (DE); Reich, Hans-Bernd, 67141 Neuhofen (DE)

(57) **Zusammenfassung**

Verwendung einer wäßrigen Zusammensetzung, enthaltend
a) ein Polyoxyethylen-polyoxypropylen-copolymer A mit einem mittleren Molekulargewicht (MGw) von über 8000 bis 20 000 und
b) ein Polyoxyethylen-polyoxyproylen-copolymer B mit einem mittleren Molekulargewicht (MGw) von 4000 bis 8000
zur Herstellung von Arzneimitteln, die subkutan oder intramuskulär verabreicht werden.

## Beschreibung

Die Erfindung betrifft die Verwendung von wäßrigen Zusammensetzungen, die zwei unterschiedliche Polyoxyethylen-polyoxypropylen-copolymere enthalten.

Wäßrige Zusammensetzungen, die Polyoxyethylen-polyoxypropylencopolymere enthalten, sind bekannt (J. Pharmac. Sci., 80, 3, 1991, S. 280 - 283, J. Colloid Interface Sci. 1990, 1997, 2, 307 - 312).

Aus der US 3,740,421 sind wäßrige Gele, die Polyoxyethylen-polyoxypropylen enthalten, bekannt.

Diese Polymere werden z.B. als Verdicker, Gelbildner oder Lösungsvermittler insbesondere für pharmazeutische und kosmetische Zubereitungen eingesetzt (Polymer Controlled Drug Delivery, 1991, 189-214, Controlled Release Society).

Aus der CA 1,072,413 sind Poloxamer-Gel-Systeme, die unterschiedliche Polyoxyethylen-polyoxypropylen-copolymere enthalten für die Anwendung in der Pharmazie und Kosmetik bekannt.

Es bestand die Aufgabe, wäßrige Zusammensetzungen zu entwickeln, die an definierten Wirkorten eine kontrollierte Abgabe eines Arzneimittels ermöglichen und einfach mit einer Spritze zu verabreichen sind.

Diese Aufgabe wird erfindungsgemäß gelöst durch die Verwendung einer wäßrigen Zusammensetzung, enthaltend
a) ein Polyoxyethylen-polyoxypropylen-copolymer A mit einem mittleren Molekulargewicht (MG_{w}) von über 8000 bis 20 000 und
b) ein Polyoxyethylen-polyoxypropylen-copolymer B mit einem mittleren Molekulargewicht (MG_{w}) von 4000 bis 8000
zur Herstellung von Arzneimitteln, die subkutan oder intramuskulär verabreicht werden.

Die erfindungsgemäß verwendeten Zusammensetzungen lassen sich bei Raumtemperatur in flüssiger Form intramuskulär und subkutan verabreichen und bilden bei Körpertemperatur ein Depotgel, aus dem Wirkstoffe kontrolliert freigesetzt werden. In den Arzneimittelformulierungen wirken die erfindungsgemäß verwendeten Zusammensetzungen aber nicht nur als Gelbildner, sondern auch als Lösungsvermittler, Emulgator und Dispergiermittel.

Die in den erfindungsgemäß verwendeten Zusammensetzungen enthaltenen Copolymere A weisen bevorzugt ein MG_{w} (mittleres Gewichtsmolekulargewicht) von 9000 bis 15 000, die Copolymere B von 5000 bis 7000 auf. Die MG-Werte sind aus den OH-Zahlen berechnet.

Die erfindungsgemäß verwendeten Polyoxyethylen-polyoxypropylencopolymere lassen sich in erster Näherung durch die folgende allgemeine Formel darstellen: 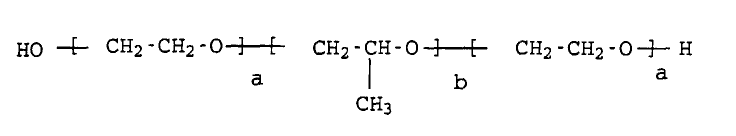 wobei a und b jeweils solche Werte bedeuten, daß die oben angegebenen Molekulargewichte erreicht werden.

Der gewichtsmäßige Anteil der Polyoxyethyleneinheiten ⁅CH₂-CH₂-O⁆ im Copolymer A beträgt bevorzugt 60 bis 80 %, insbesondere 70-76 %; im Copolymer B bevorzugt 70 bis 90 %, insbesondere 77 bis 84 %. Das Verhältnis der Werte a und b ist jeweils entsprechend eingestellt.

Die Copolymere A und B sind an sich bekannte Polymere (Handelsprodukte) und werden auf bekannte Weise hergestellt. Die Copolymere können zur Stabilisierung BHT enthalten und liegen üblicherweise als grobkörnige Pulver von wachsähnlicher Konsistenz vor. Sie werden allgemein auch als "Poloxamere" bezeichnet.

Die erfindungsgemäß verwendeten Zusammensetzungen enthalten die Copolymere A bevorzugt in Mengen von 5 bis 30, insbesondere 10 bis 25 Gewichtsprozent und die Copolymere B bevorzugt in Mengen von 1 bis 30, insbesondere 2 bis 25 Gewichtsprozent, jeweils bezogen auf die Gesamtzusammensetzung.

Die Zusammensetzungen weisen den Vorteil auf, daß sich die Sol-Gel-Übergangstemperatur maßgeschneidert, insbesondere im Bereich von 20°C bis 40°C, einstellen läßt. Darüberhinaus kann die Viskosität des entstehenden Gels eingestellt werden. Außerdem ist zu beobachten, daß die Zusammensetzungen über einen weiten Temperaturbereich (30-90°C) mit steigenden Temperaturen keinen Viskositätsabfall zeigen.

Die erfindungsgemäß verwendeten Zusammensetzungen lassen sich im allgemeinen auf zwei Wegen herstellen:

Beim "Kaltverfahren" werden die Copolymere zusammen oder nacheinander in Wasser von 0-25°C eingerührt. Unlösliche Bestandteile können in einem organischen Lösungsmittel wie Ethanol, i-Propanol oder Propylenglykol gelöst und dann mit der wässrigen Phase homogen vereinigt werden.

Beim "Heißverfahren" werden die Copolymere zusammen oder nacheinander wie oben gelöst, nur beträgt die Temperatur 60-100°C und die Lösung wird anschließend auf 25°C abgekühlt.

Die erfindungsgemäß verwendeten Zusammensetzungen können neben den genannten Polymeren und Lösungsmitteln noch die entsprechenden pharmazeutischen oder kosmetischen Wirkstoffe enthalten. Die entsprechenden Wirkstoffe können als Lösung beigemischt werden.

Für temperaturempfindliche Wirkstoffe ist selbstverständlich das Kaltverfahren bevorzugt.

Die erfindungsgemäß verwendeten Zusammensetzungen können zusätzlich 0,01 bis 5, bevorzugt 0,02 bis 1 Gewichtsprozent Polyacrylsäure enthalten. Die Polyacrylsäure führt zu einer Erniedrigung der Gelierungstemperatur und Erhöhung der Viskosität.

Als Beispiele für pharmazeutische Wirkstoffe seien genannt:
Aldosteron Antagonisten, wie z.B. Kaliumcanrenoat, Spironolacton und Furosemid;
Analgetika/Antirheumatika, wie z.B. Diclofenac, Ibuprofen, Proxicam, Tramadol und Opiate;
Antiarrhythmika, wie beispielsweise Mexiletin, Propafenon oder Sotalol;
Antibiotika/Chemotherapeutika wie z.B. Penicilline, Cephalosporine, Tetracycline, Aminoglykoside, Chloramphenicol, Thiamphenicol, Lincomycine und Makrolid-Antibiotika, Polypeptid-Antibiotika, Chinolone, Nitroimidazole, Malariamittel, Virusstatika, Enzyminhibitoren z.B. β-Lactamase;
Antiepileptika, z.B. Phenobarbital, Carbamazepin;
Antifibrinolytika, wie z. B. Tranexamsäure, Aminomethylbenzoesäure;
Antihypertonika, z.B. Clonidin, Prazosin, Minoxidil, Verapamil oder Nifedipin;
Antikoagulantia, beispielsweise Phenprocoumon oder Heparin;
Antihypotonika;
Antimykotika, z.B. Miconazol, Itraconazol, Flucytosin, Amphotericin B, Nystatin oder Griseofulvin;
Beta-Rezeptorenblocker, Calciumantagonisten und ACE-Hemmer wie u.a. Atenolol, Propranolol, Metoprolol, Nifedepin, Nitrendipin, Nisoldipin, Dilthiazem, Captopril, Lisinopril, Enalapril oder Ramipril;
Antiasthmatika, u.a. Terbutaliri, Salbutanmol, Theophyllin;
Diuretika, beispielsweise Piretanid, Furosemid, Hydrochlorothiazid oder Chlortalidon;
Enzympräparate und Transportproteine wie u.a. Aprotinin, Hyaluronidase, Kallidinogenase und L-Carnitin;
Fibrinolytika, wie Alteplase, Urokinase, Streptokinase und andere;
Gynäkologika, Kontrazeptiva, Prostaglandine und ihre Derivate;
Hypophysen-, Hypothalamushormone, andere regulatorische Peptide und ihre Hemmstoffe sowie Derivate der genannten Hormone;
Immuntherapeutika und Zytokine z.B. Interferone;
Kardiaka und Koronarmittel, z.B. Glyceroltrinitrat, Isosorbidmononitrat, -dinitrat;
Lokalanästhetika und Neuraltherapeutika, beispielsweise Mepicacain, Bupivacain, Lidocain, Procain, Tetracain und andere;
Nebenschilddrüsenhormone/Calciumstoffwechselregulatoren;
Parkinsonmittel und Psychopharmaka wie z. B. Amitryptylin, Doxepin, Benzodiazepine;
Schilddrüsentherapeutika; Sera, Immunglobuline; Sexualhormone und ihre Hemmstoffe bzw deren Analoga; Thrombozytenaggregationshemmer; Zytostatika und Metastasehemmer, wie z. B. Alkylantien, Antimetabolite, Alkaioide, Cisplatin, Interferone, LH-RH- Analoga;
Lipidsenker, wie z. B. Fenofibrat, Bezafibrat, Simvastatin.

Diese Wirkstoffe können die Viskositätseigenschaften der Zusammensetzungen beeinflussen. So führt z.B. Ibuprofen zu einer Erniedrigung der Gelierungstemperatur.

Die erfindungsgemäß verwendeten Zusammensetzungen können auch noch Salze wie NaCl oder KCl enthalten, die gegebenenfalls eine Viskositätserhöhung bewirken. Derartige Salze können in Mengen von 1-20 Gew.-% vorhanden sein.

Eine erfindungsgemäß verwendete Zusammensetzung kann demnach z.B. folgende Komponenten enthalten:

| | |
|---|---|
| Wirkstoff | x g |
| Propylenglykol*) | 20 g |
| Copolymer A | 22 g |
| Copolymer B | 5 g |
| NaCl | 1 g |
| H₂O | ad 100 g |

| | |
|---|---|
| *) Lösungsvermittler im Falle von schwer wasserlöslichen Wirkstoffen | |

Darüberhinaus können die erfindungsgemäß verwendeten Zusammensetzungen noch die üblichen Hilfsstoffe für pharmazeutische Zubereitungen enthalten, wie z.B. Tenside.

### Beispiele 1-8

Ein Polyoxyethylen-polyoxypropylen-copolymer mit einem mittleren MG_{w} von 12 000 g/mol und einem gewichtsmäßigen Anteil an Polyoxyethylen von 73% (Lutrol® F 127, BASF) wurde bei 5°C in H₂O durch Rühren aufgelöst. Anschließend wurde ein Polyoxyethylen-polyoxypropylen-copolymer mit einem mittleren MGw von 6000 g/mol und einem gewichtsmäßigen Anteil an Polyoxyethylen von 80% (Lutrol^{R} F68, BASF) ebenfalls bei 5°C unter Rühren zugegeben, bis eine Lösung erhalten wurde.

Tabelle 1 enthält die Angaben zu den in den einzelnen Beispielen jeweils eingesetzten Mengen, wobei es sich bei Beispiel A um ein Vergleichsbeispiel handelt (nur ein Copolymer A in der Zusammensetzung).

| | **Copolymer A** | **Copolymer B** |
|---|---|---|
| A | 20 | -- |
| 1 | 20 | 5 |
| 2 | 20 | 10 |
| 3 | 20 | 20 |
| 4 | 17,5 | 10 |
| 5 | 17,5 | 15 |
| 6 | 15 | 10 |
| 7 | 15 | 20 |
| 8 | 12,5 | 20 |

### Beispiele 9-14

Wie für die Beispiele 1-8 wurden wässrige Zubereitungen hergestellt, die die in Tabelle 2 angegebene Zusammensetzung aufweisen.

Die Polyacrylsäure (Carbopol® 940, BF Goodrich) wurde in einem Aliquot H₂O dispergiert und darin bei 5°C mit der Lösung der Copolymere A und B vereinigt. Die Polyacrylsäure wurde dann mit Triethanolamin neutralisiert.

**Tabelle 2**

| **Beispiel** | **Copolymer A** | **Copolymer B** | **PAA** |
|---|---|---|---|
| 9 | 12,5 | 20 | 0,05 |
| 10 | 12,5 | 20 | 0,1 |
| 11 | 15 | 20 | 0,1 |
| 12 | 15 | 20 | 0,25 |
| 13 | 17,5 | 15 | 0,1 |
| 14 | 12,5 | 15 | 0,1 |

### Beispiele 15 und 16

Es wurden Zusammensetzungen wie oben beschrieben hergestellt. Ibuprofen wurde in Ethanol/Propylenglykol gelöst und diese Lösung der Lösung der Polymere zugefügt (s. Tabelle 3).

**Tabelle 3**

| **Beispiel** | **Copolymer A** | **Copolymer B** | **PAA** | **Ibuprofen** |
|---|---|---|---|---|
| 15 | 12,5 | 15 | -- | 5 |
| 16 | 12,5 | 15 | 0,1 | 5 |

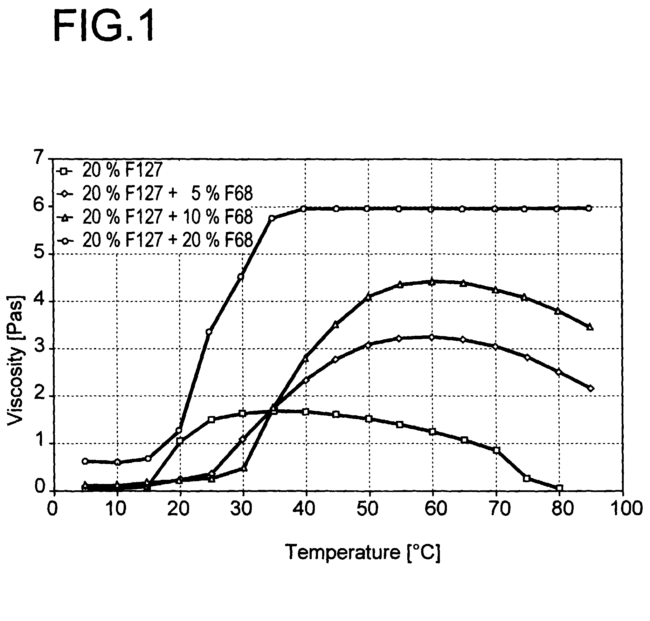

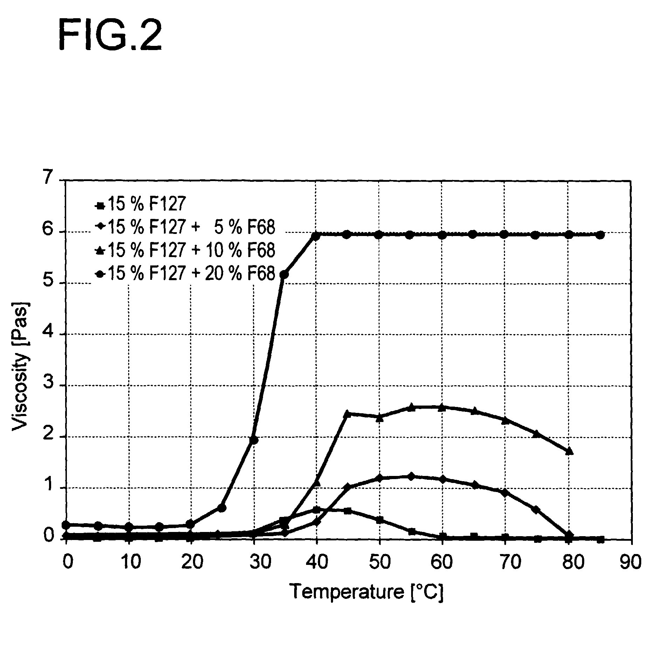

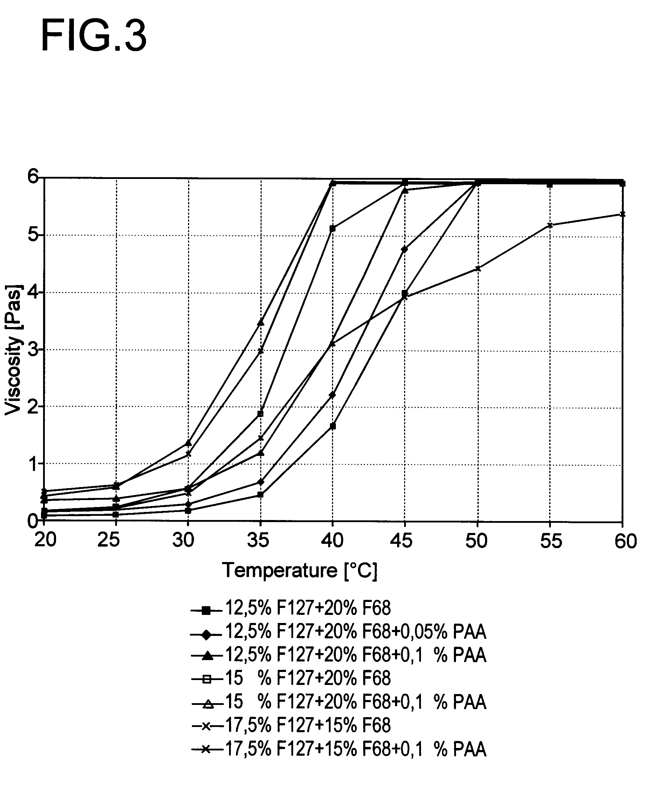

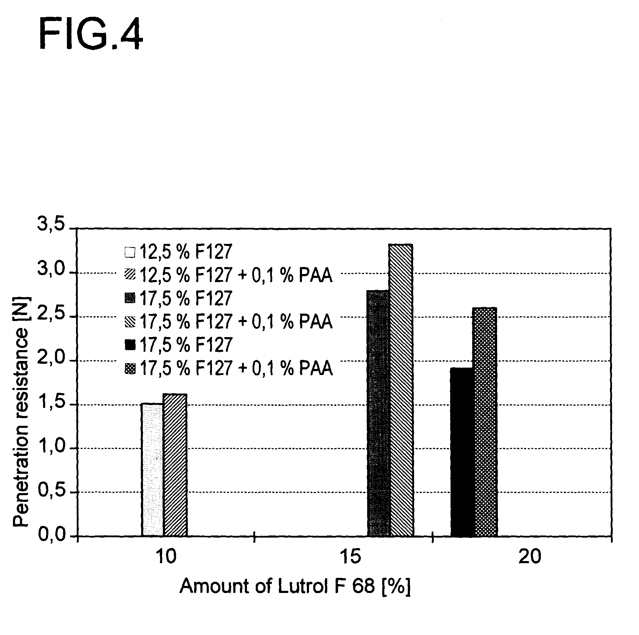

Die Ergebnisse mit den Zusammensetzungen nach Vergleichsbeispiel A und den Beispielen 1 bis 16 sind an den Figuren 1 bis 4 zu entnehmen.

Figuren 1 und 2 zeigen die Abhängigkeit der Viskosität und die Sol-Gel-Übergangstemperatur von den Konzentrationen der Polymere in den Zusammensetzungen.

Figur 3 zeigt den Einfluß der zugeführten Mengen PAA auf die Viskosität und Figur 4 zeigt die Gelfestigkeit für bestimmte Zusammensetzungen.

Die Viskosität wurde dabei mit einem Viskosimeter der Firma Physika Meßtechnik GmbH (Rheolab MC 10) gemessen. Die Temperatur wurde mit einer Aufheizungsrate von 1°C pro Minute erhöht, die Rotationsgeschwindigkeit betrug 250 rpm. Die Gelfestigkeit (Penetrationresistance) wurde bei 40°C mit einem Software kontrollierten Penetrometer (TA-XTL Texture Analyzer, Firma Stable Micro-Systems) mit einer 5 kg beladenen Zelle und einer 20 mm-Probe gemessen. Die Testgeschwindigkeit betrug 1 mm/s, die Eindringtiefe 5 mm.

Die so hergestellten Zusammensetzungen können in an sich bekannter Weise mittels einer Spritze subkutan oder intramuskulär verabreicht werden. Damit kann unter der Haut z.B. in der Nähe einer entzündeten Stelle, oder im Muskel ein Arzneimitteldepot erhalten werden, das, da die Zusammensetzung bei Körpertemperatur geliert, das Arzneimittel kontrolliert freigeben kann.

## Patentansprüche

1. Verwendung einer wäßrigen Zusammensetzung, enthaltend
a) ein Polyoxyethylen-polyoxypropylen-copolymer A mit einem mittleren Molekulargewicht (MG_{w}) von über 8000 bis 20 000 und
b) ein Polyoxyethylen-polyoxypropylen-copolymer B mit einem mittleren Molekulargewicht (MG_{w}) von 4000 bis 8000
zur Herstellung von Arzneimitteln, die subkutan oder intramuskulär verabreicht werden.

2. Verwendung nach Anspruch 1, dadurch gekennzeichnet, daß Copolymer A ein MG_{w} von 9000 bis 15 000 und Copolymer B ein MG_{w} von 5000 bis 7000 aufweist.

3. Verwendung nach Anspruch 1, dadurch gekennzeichnet, daß Copolymer A einen gewichtsmäßigen Anteil an Polyoxyethyleneinheiten -CH₂-CH₂-O- von 60 bis 80 % und Copolymer B einen solchen Anteil von 70 bis 90 % aufweist.

4. Verwendung nach Anspruch 1, dadurch gekennzeichnet, daß die Zusammensetzung 5 bis 30 Gewichtsprozent Copolymer A und 1 bis 30 Gewichtsprozent Copolymer B enthält.

5. Verwendung nach Anspruch 1, dadurch gekennzeichnet, daß die Zusammensetzung zusätzlich 0,01 bis 5 Gewichtsprozent Polyacrylsäure enthält.

6. Verwendung nach Anspruch 1, dadurch gekennzeichnet, daß die Zusammensetzung eine Sol-Gel-Übergangstemperatur von 20°C bis 40°C aufweist.
